# EUROPEAN PATENT SPECIFICATION

(11) **EP 2 967 159 B1**
(45) Date of publication and mention of the grant of the patent: **01.05.2019**
(21) Application number: 14769690.0
(22) Date of filing: 14.03.2014
(51) Int. Cl.: A41B 11/02, A63B 71/12

(54) **PROTECTIVE ANKLE AND CALF SLEEVE**
SCHÜTZENDE KNÖCHEL- UND WADENMANSCHETTE
MANCHON DE PROTECTION DE CHEVILLE ET DE MOLLET

(30) Priority: 15.03.2013 US 201361792278 P; 11.12.2013 US 201361914736 P; 13.03.2014 US 201414208782
(43) Date of publication of application: 20.01.2016
(73) Proprietor: NIKE Innovate C.V., Beaverton, OR 97005-6453 (US)
(72) Inventor: BEHREND, Carl, Beaverton, Oregon 97005-6453 (US); MCLACHLAN, Oliver, Beaverton, Oregon 97005-6453 (US); MORRISON, Catherine F., Beaverton, Oregon 97005-6453 (US)
(74) Representative: Beattie, Alex Thomas Stewart
(86) International application number: PCT/US2014/027266
(87) International publication number: WO 2014/152372

(56) References cited:
- WO-A2-01/87432
- WO-A2-2007/036751
- CN-Y- 2 628 105
- GB-A- 2 328 859
- GB-A- 2 450 525
- US-A- 4 756 026
- US-A1- 2003 191 420
- US-A1- 2011 296 588
- US-A1- 2012 102 613

## Description

### FIELD OF THE INVENTION

Aspects hereof relate to protective gear. More particularly, an ankle sleeve, or ankle and calf sleeve, is disclosed for use by an athlete participating in or practicing sports, such as soccer, football, etc., to prevent injuries and to provide support to the athlete's legs around the ankles.

### BACKGROUND OF THE INVENTION

Athletes who practice impact sports like soccer are prone to injuries and, therefore, require extra protective gear, particularly for the lower extremities. The protective gear used, although very necessary, can be heavy and/or uncomfortable when worn for long periods of time. As such, athletes may be prone not to wear their protective gear during practice sessions. However, athletes are still prone to injuries during practice sessions, a risk that may be exacerbated if an athlete chooses not to wear protective gear during practice.

Therefore, there is a need for light and comfortable protective gear that can be regularly worn while still providing a degree of protection for the athlete.

US4756026 discloses contact sport protection sleeves and pads for the forearm, elbow, knee and shin of the players including at least one protection panel of flexible elastomeric polymeric foam or of rigid plastic attached to an elastomeric sleeve with two flexible elastomeric polymeric foam edge pads abutting the lengthwise edges of the protection panel with the panel and edge pads all enveloped in elastomeric fabric with stitching between the abutting of the panel and the two edge pads providing extra protection with flexibility to reduce any restriction of movement of the player.
WO 01/87432 discloses a foot and ankle protective garment which discloses multiple cushioning elements having a slit or distance between each other.

### SUMMARY OF THE INVENTION

Aspects hereof generally relate to protective gear for an ankle and/or shin area of an athlete's or user's legs (hereinafter, athlete and user will be used interchangeably). The protective gear comprises a sleeve that features special padded regions for optimal protection without compromising comfort. The ankle sleeve may be knit from synthetic fibers, natural fibers, or a blend of synthetic and natural fibers. Thus, the disclosed protective gear is comfortable and can be worn on a regular basis for prolonged periods of time, increasing the user's safety in all instances, such as, for example, practice and actual game play.

The protective gear features special padding at strategic locations to offer added protection without compromising comfort.

The main body of the protective gear may be knit, and in particular may be made using a circular knit, into a sleeve so that the final product has no uncomfortable seams pressing down on the ankle or foot of a user. The sleeve may be knit from a combination of different types of yarns to impart different physical properties to the sleeve. For example, elastic yarns may be employed to knit an elastic sleeve that imparts higher compression strength on the ankle and foot than a regular knitted sock. The desired level of compression may be adjusted. For example, the protective gear may be more compressive than a regular sock but less compressive than a medical sleeve. Alternatively, the sleeve may be made from woven materials with strategic stitch or sewing lines arranged for the comfort of the athlete.

The ankle sleeve may be designed to cover at least a portion of the foot and at least a portion around the ankle area of a user. The ankle sleeve may also be designed with a system for extending the protected region from just the lower shin to the whole shin, for example, all the way up to a region just below the knee.

The cushioning used for padding may comprise an impact-resistant foam such as a polyurethane foam. The foam density may be uniform throughout all the cushioned regions or may be varied in the different cushioned regions.

Some or all cushioning pads may feature an extra protective layer to provide abrasion and/or laceration protection. The extra protective layer may comprise a rubbery piece and/or a hard plastic shell over the cushioning pads. Alternatively or additionally, a protective layer may provide protection from abrasion and/or laceration without an underlying pad to provide cushioning.

Additional objects, advantages, and novel features will be set forth in part in the description that follows, and in part will become apparent to those skilled in the art upon examination of the following, or may be learned by practice of the technology.

### BRIEF DESCRIPTION OF THE DRAWING

Aspects hereof are described in detail below with reference to the attached drawing figures, wherein:
FIG. 1A is a back view of a piece of protective gear as worn;
FIG. 1B is a lateral view of the protective gear of FIG. 1A;
FIG. 1C is a lateral and partial front view of the protective gear of FIG. 1A;
FIG. 1D is a front view of the protective gear of FIG. 1A;
FIG. 1E is a medial and partial front view of the protective gear of FIG. 1A;
FIG. 1F is a medial view of the protective gear of FIG. 1A;
FIG. 2A is a close-up view of FIG. IF;
FIG. 2B is a cross section of a region in FIG. 2A showing an exemplary cushioning structure;
FIG. 3 is a front view of the protective gear featuring an exemplary adaptor element for removably attaching a full-length shin guard;
FIG. 4 is a lateral view of an alternative arrangement of a calf and ankle sleeve;
FIG. 5 is a view similar to FIG. 4, showing a medial view;
FIG. 6 is a view similar to FIGS. 4 and 5, showing a front view;
FIG. 7 is a lateral view of an alternative arrangement of an ankle sleeve;
FIG. 8 is a view similar to FIG. 7, showing a medial view; and
FIG. 9 is an exploded view showing the layering of materials used in portions of the sleeves of FIGS. 4-8.

### DETAILED DESCRIPTION OF THE INVENTION

The subject matter of certain aspects is described with specificity herein to meet statutory requirements. However, the description itself is not intended to limit the scope of this patent. Rather, the inventors have contemplated that the claimed subject matter might also be embodied in other ways, to include different elements or combinations of elements similar to the ones described in this document, in conjunction with other present or future technologies.

Aspects hereof relate to protective gear that is wearable by athletes of high impact sports that require protection in the foot and ankle area. The protective gear includes a plurality of cushioning pads located at strategic locations to provide optimal protection against potential injuries from impact to the ankle and foot area of an athlete of sports like, for example, soccer.

Accordingly, in one aspect, protective gear is disclosed for an ankle and foot of an athlete that is comfortable enough to be used regularly and at the same time provides the support and protection needed for preventing injuries. More particularly, the protective gear, when used, is effective in preventing high impact injuries such as trauma.

To maximize comfort, the main body of the protective gear comprises a knitted sleeve. The knitted sleeve may be knitted using a circular knitting technique such that no seams will compromise the comfort of the knitted sleeve. The knitted sleeve may be structured generally to fit and cover at least a portion of the foot and ankle of an athlete. In one example, the sleeve may be knitted continuously like a sock, covering the whole foot, toes, and ankle of an athlete when worn. In another example, the sleeve may be knit like a sock without the toe portion, and in yet another different example, the sleeve may be knit without a toe and heel portion (as generally shown in the drawings). The length of the knitted sleeve may be adjusted according to the area of protection desired, particularly for the shin of a leg of an athlete.

The knitted sleeve can be knitted from a combination of different types of yarns, such as elastic yarns for providing elasticity, moisture management yarns for controlling moisture build up in the sleeve when worn, high resilience yarns to provide resiliency, natural fiber yarns to provide softness and comfort, etc. Examples of elastic yarns are, for example, elastane or spandex yarns; examples of resilient yarns may include, for example, Kevlar® yarns; and moisture management yarns may be synthetic yarns that have wicking properties or yarns treated with moisture management coatings that have the properties of wicking moisture away from the skin. A knitted sleeve, in accordance with the present invention, may be formed from natural fibers such as cotton, from synthetic fibers such as polyester or nylon (or examples listed above), and/or from a mixture or blend of such fibers. Moreover, different types of fibers and/or different blends of fibers may be used at different locations on a knitted sleeve.

The amount of each type of yarn, and more particularly, the amount of the elastic yarns, may be adjusted such that the sleeve may provide compressive support to the ankle and foot of an athlete when worn. The amount of elasticity may be especially controlled such that the amount of compression offered by the sleeve may be higher than a regular sock but lower than a medical compression sleeve.

In another aspect, protective gear is disclosed for the ankle and foot area of an athlete further comprising cushioning pads in strategic areas of the outer surface of the knitted sleeve. The cushioning pads can be made of materials that absorb impact. Such impact-absorbing materials may be, for example, foam materials, such as polyurethane foams, polystyrene foams, Styrofoam, etc. The foam material density and thickness may be uniform throughout the protective gear or may be varied at different regions of the protective gear. Also, different combinations of foam materials may be used at different areas of the protective gear, depending on the type and amount of protection desired. For example, a foam used for cushioning pads protecting bony areas may be denser than the foam used for cushioning pads protecting muscular areas. The thickness of the foam may alternatively or additionally be varied for cushioning pads in different regions, depending on the amount of impact resistance desired.

In different aspects, a thermoplastic elastomer (TPE) may be used for the cushioning pads instead of foam materials. The TPE material used, like the foam materials, may be provided with different thickness and density according to the different cushioning pads provided to different areas of the protective gear.

Other materials suitable for the formation of the cushioning pads may be, for example, a lofted knit structure or a spacer mesh structure. All the materials presented above and any other material suitable for the formation of pads may be used. Further, a combination of different materials may be used for forming each individual pad, or different materials may be used at different regions in the protective gear providing a more customizable protective profile to the protective gear.

The cushioning pads may be affixed to the outer surface of the knitted sleeve at, for example, areas corresponding to an Achilles tendon, a medial malleolus, a lateral malleolus, and a lower shin area. The cushioning pads may be affixed onto the knitted sleeve by, for example, securing an overlaying textile or other sheet-type material to the knitted sleeve over one or more cushioning pads. An overlaying textile or sheet-type material may be secured to the knitted sleeve by sewing around a perimeter of each cushioning pad, by applying a hot melt adhesive around a perimeter of each cushioning pad, or by other means. For example, cushioning pads may also be affixed onto the outer surface of the knitted sleeve by applying an ultrasonically activated adhesive around the edge of each pad. The cushioning pads may also be inserted into pockets formed from the same material as the knitted sleeve, or the pockets may be formed from a different material having at least the same stretchability as the knitted sleeve on the surface of the knitted sleeve. After insertion of the cushioning pads into the pockets, the pockets may be subsequently sealed. The pads may also be affixed onto the knitted sleeve by placing an adhesive around the perimeter of each pad, covering the pads with the same material or with a different material that is at least as stretchy as the knitted sleeve, and finally, activating the adhesive so that the pads may be fixed in place without applying any adhesive on the cushioning pads themselves. The "pocket" and the "cover and adhesive" techniques will provide a layer of protection to the pads. Alternatively, any other method suitable for affixing the cushioning pads onto the surface of the knitted sleeve may be employed. Pads may be secured directly to the knit sleeve using an adhesive, if desired.

Depending on the materials used for the cushioning pads, the pads may be as flexible as the knitted sleeve, or the pads may exhibit lower or limited flexibility, which may make the sleeve difficult to wear if the pads are not appropriately positioned and/or configured. Therefore, each cushioning pad in the protective gear may have a shape that is specifically designed for the area that it is meant to protect when worn, as well as to facilitate the donning of the knitted sleeve. As such, the cushioning pads may feature shapes optimal for protection of each of the protected areas, such as the Achilles tendon, the medial malleolus, the lateral malleolus, and the lower shin area of an athlete's foot. Donning of the sleeve may be facilitated by the location and/or geometry of the cushioning pads. For example, strategic slits may be provided in the cushioning pads for easier donning. For example, the pad affixed to the area on the knitted sleeve corresponding to the Achilles tendon, may be a narrow and elongated cushioning pad, thereby preventing the Achilles tendon pad from interfering with donning. The cushioning pad affixed to the area of the lower shin area may be wide enough to cover most of the lower shin and may feature a slit that is at least one-third of the length of the pad to facilitate easy donning of the sleeve. The slit may have various configurations, such as curvilinear, intersecting slits, angled slits, and the like, to facilitate donning in accordance with the present invention. In other areas, such as the medial malleolus, there may be more than one pad covering the area so that the sleeve can be easily donned by providing a stretchable padded area without compromising the coverage provided by the pad or pads. Alternatively, a cushioning pad may have a stretch zone that provides somewhat reduced protection but permits greater stretchability for donning.

In yet another aspect, at least one cushioning pad of the protective gear may additionally have a rubbery and/or hard shell to protect the pad against abrasion. The shell may be urethane based, silicone based, polyethylene based, etc. More specifically, the shell may be made of, for example, a thermoplastic polyurethane, a silicone plastic, etc.

Therefore, aspects look to provide at least some of the advantages of protective gear for protection of an ankle and foot of an athlete (e.g., comfort, energy distribution, and energy absorption) while reducing some of the disadvantages associated with traditional protective gear.

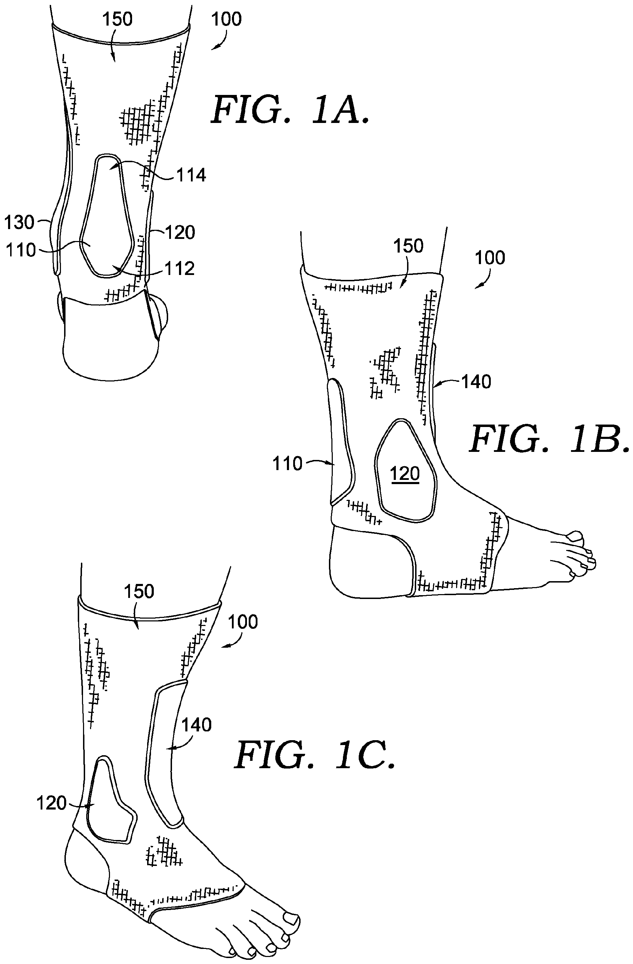

FIGS. 1A-1F illustrate different views of an exemplary protective gear 100. As shown in FIGS. 1A-1F, the protective gear 100 comprises an elastic knitted sleeve 150 and cushioning pads 110, 120, 130, and 140. Cushioning pads 110, 120, 130, and 140 may be placed in alignment with the lateral and medial malleolus regions, the Achilles tendon, and the lower shin area of a user's leg, and may be provided above the area where a shoe collar would be so that the cushioning pads 110, 120, 130, and 140 will not interfere with the shoe collar when the protective gear 100 is worn with shoes.

The knitted sleeve 150 in FIGS. 1A-1F is depicted as covering at least a portion of the foot of a user, not including the toes or the heel. However, the knitted sleeve 150 may, in other examples, be knitted as a sock covering the ankle and the heel but not the toes, covering the ankle and the toes but not the heel, covering the whole foot and ankle of a user, or yet in a different example, the knitted sleeve 150 may be knitted as a sock-glove, having individual sheaths for each toe. Additionally, depending on the elastic and physical properties desired for the knitted sleeve 150, different regions of the sleeve may be knitted with different types of yarn or combinations of yarn, having different elastic and physical properties.

Turning now to each figure, FIG. 1A depicts a back view of the protective gear 100 in an as-worn position on a foot and ankle of a user. FIG. 1A mainly shows a cushioning pad 110 provided for protection of an Achilles tendon of a user. As seen in FIG. 1A, the Achilles tendon cushioning pad 110 is placed right above the Achilles tendon of the user and is organically shaped to generally conform to the leg of the user in such a way that it provides a wider protection in the lower portion 112 of the Achilles tendon cushioning pad 110 and slowly narrows towards the top portion 114 of the Achilles tendon cushioning pad 110. However, the Achilles tendon cushioning pad 110 may also be geometrically shaped such as an elongated straight-edged rectangle, an elongated rounded-edged rectangle, etc. The Achilles tendon cushioning pad 110 may optionally have a protective shell to provide added abrasion/laceration protection, with or without a foam pad.

FIG. 1B is a lateral view of the protective gear 100, showing the alignment of cushioning pads 110, 120, and 140 in an as-worn position on a foot and ankle of a user. In FIG. 1B, a front view of the cushioning pad 120 corresponding to the lateral malleolus is shown. The lateral malleolus cushioning pad 120 may comprise a single pad organically shaped to cover the whole lateral malleolus (as shown), or alternatively, the lateral malleolus cushioning pad 120 may comprise more than one cushioning pad in order to aid in the donning of the protective gear 100 by not limiting the stretchability of the knitted sleeve 150. As in the case of Achilles tendon cushioning pad 110, lateral malleolus cushioning pad 120 (or pads), may optionally further comprise a protective shell to protect the lateral malleolus cushioning pad 120 against abrasion, either alone or in combination with a foam pad.

FIG. 1C is a lateral and partial front view of the protective gear 100. In this view, the alignment of the lateral malleolus cushioning pad 120 and the lower shin cushioning pad 140 can be observed.

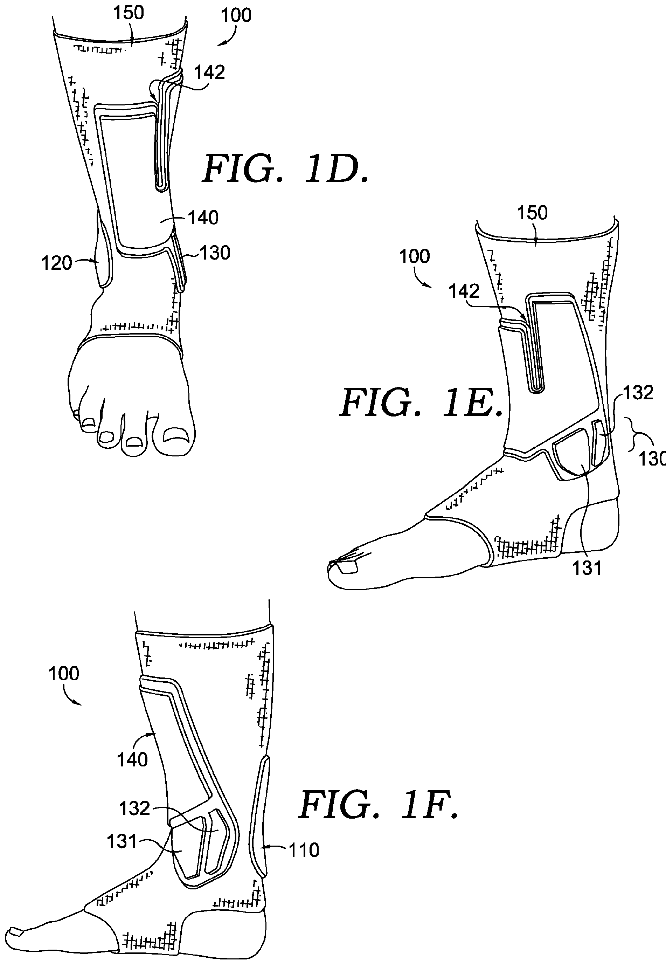

FIG. 1D is a front view of the protective gear 100. The relative alignment of the lateral and medial malleolus cushioning pads 120 and 130, respectively, with the lower shin cushioning pad 140, as well as a detailed view of the lower shin cushioning pad 140 can be observed. Lower shin cushioning pad 140 may comprise the largest protective cushioning pad of the protective gear 100. The lower shin cushioning pad 140 may cover at least the front lower area of the user's leg that aligns with the medial and lateral malleolus of the user, up to the height of the knitted sleeve 150. Because of its large size, the elasticity of the knitted sleeve 150 may potentially be limited by the elasticity of the lower shin cushioning pad 140. As such, lower shin cushioning pad 140 is provided with a slit 142 to facilitate donning of the protective gear 100. Slit 142 may be linear, curvilinear, or any other shape. Further, slit 142 may comprise multiple slits. In the example shown in FIG. ID, slit 142 has a length that is at least 30% of the total length of the lower shin cushioning pad 140. The slit 142 allows the knitted sleeve 150 to stretch beyond the stretchability of lower shin cushioning pad 140 to an open position, thereby providing easy donning of the protective gear 100. Because of the elastic properties of the knitted sleeve 150, after donning of the protective gear 100 onto the user's foot and ankle, the slit 142 returns to its starting position and substantially closed, thereby offering a nearly continuous protective pad that will protect the lower shin area of the user. Lower shin cushioning pad 140 may optionally further comprise a protective shell over all or part of protective cushioning pad 140.

FIG. 1E is a medial and partial front view of the protective gear 100. In this view, the alignment of the medial malleolus cushioning pad 130 and the lower shin cushioning pad 140 can be observed. The medial malleolus cushioning structure 130 is depicted as being comprised of two medial cushioning pads 131 and 132; however, the medial cushioning structure 130 may be comprised of only one cushioning pad or more than two cushioning pads.

In the example shown in the figures, the medial cushioning pads 131 and 132 have distinct functions. In particular, the medial cushioning pad 132 is located behind the medial malleolus to protect the bone from stud trauma. Medial cushioning pad 131 may optionally further comprise a protective shell, as may medial cushioning pad 132.

FIG. IF is a medial view of the protective gear 100. The alignment of the medial cushioning pads 131 and 132, the lower shin cushioning pad 140, and the Achilles tendon cushioning pad 110 are shown. As seen, the pads are placed in a position that will not interfere with the collar of a shoe, when shoes are worn by the user.

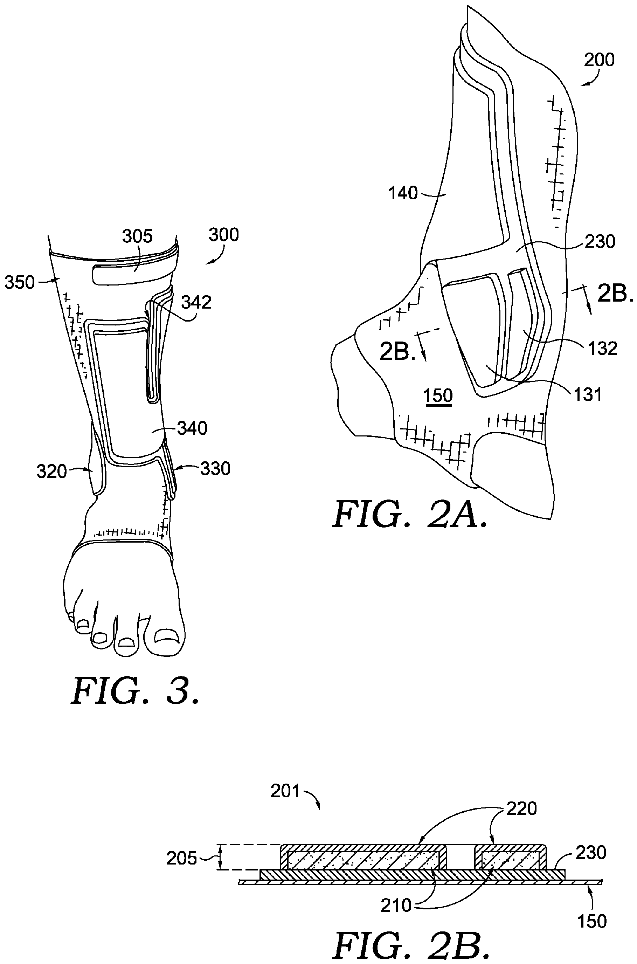

FIG. 2A is a close-up view 200 of cushioning structures on a protective gear, such as in FIG. IF, and FIG. 2B is a cross section 201 of a region in FIG. 2A showing an exemplary cushioning structure. As described above, the cushioning pads may additionally have a rubbery or hard shell 220 on all of the different cushioning pads 210, when affixed to the knitted sleeve 150. The cushioning pads 210 may be affixed directly onto the knitted sleeve's outer surface, or may be affixed to an intermediate surface 230. The intermediate surface 230 may be less elastic than, or as elastic as, the knitted sleeve 150 and may comprise any suitable sheet-type material for attaching the cushioning pads 210 thereto. If not on all the cushioning pads, the hard or rubbery shell 230 may be at least on the cushioning pads that are most likely to be subject to damage from abrasion or laceration, such as the medial cushioning pads 131 and 132 and the lateral malleolus cushioning pad 120 shown in FIGS. 1A-1F. In the case where there are more than one pad, such as shown for the medial malleolus cushioning structure 130, the rubbery or hard shell 230 may surround each pad (as shown in FIG. 2B), or may surround all the pads in the medial malleolus cushioning structure 130 at the same time, for example, as a dome. The total thickness 205 of a cushioning structure may be selected to provide a desired degree of protection while maintaining a desired level of comfort, mobility, and/or flexibility. For example, total thickness 205 may be between 2 mm and 6 mm, preferably total thickness may be approximately 4 mm.

FIG. 3 is a front view of the protective gear 300, featuring an exemplary adaptor element 305 for removably attaching a full-length shin guard to the protective gear 300. The adaptor element 305 may be placed on the outer surface of the knitted sleeve 350 (as shown), or may be placed on the inner surface of the knitted sleeve 350. The adaptor element may comprise a hook and loop structure, snap-on buttons, a zipper, a cuff that is designed to go over the full-length shin guard to hold it in place, a strap system, a buckle, or any other kind of adaptor that will serve to attach or hold a full-length shin guard. The different cushioning pads 320, 330, and 340, as well as an Achilles tendon pad (not shown), correspond to the cushioning pads 120, 130, 140, and 110 described in FIGS. 1A-1F. Further, a slit 342 is provided in cushioning pad 340 to facilitate donning.

While not depicted, it is contemplated that the cushioning pad 140/340, and the knitted sleeve 150/350, may be lengthened to cover the leg of a user up to a region just below the knee to provide a full-length shin protecting guard.

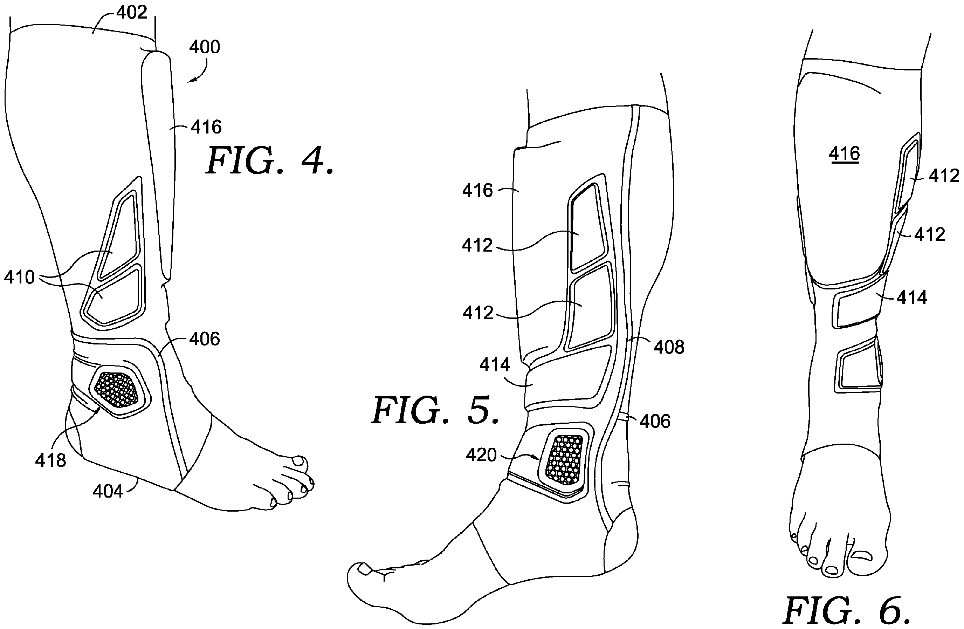

FIGS. 4-9 illustrate an alternative arrangement, not covered by the claims, showing sleeves made from woven components that are stitched together. As best seen in FIGS. 4 and 5, an ankle and calf sleeve 400 is shown that has an upper sleeve 402 and a lower sleeve 404. Sleeves 402 and 404 are coupled together by sewing along stitch lines 406 and 408. The upper sleeve 402 preferably includes lateral cushioning pads 410. As best seen in FIG. 5, upper sleeve 402 also includes medial cushioning pads 412 and transverse cushioning pad 414. Cushioning pads 410, 412, and 414 are preferably woven within the upper sleeve 402 such that their position is maintained relative to the upper sleeve 402. The pads could, alternatively, be attached to the interior or exterior of the upper sleeve 402. As best seen by examining FIGS. 4-6, the cushioning pads 410, 412, and 414 are spaced and configured to define a shin guard area 416. A separate shin guard may be placed under upper sleeve 402 located in shin guard area 416, as represented by the bulge in FIGS. 4-6 below this area. The cushioning pads 410, 412, and 414 aid in maintaining this shin guard in place. It should also be understood that a shin guard could be placed and held within upper sleeve 402, similarly to cushioning pads 410, 412, and 414.

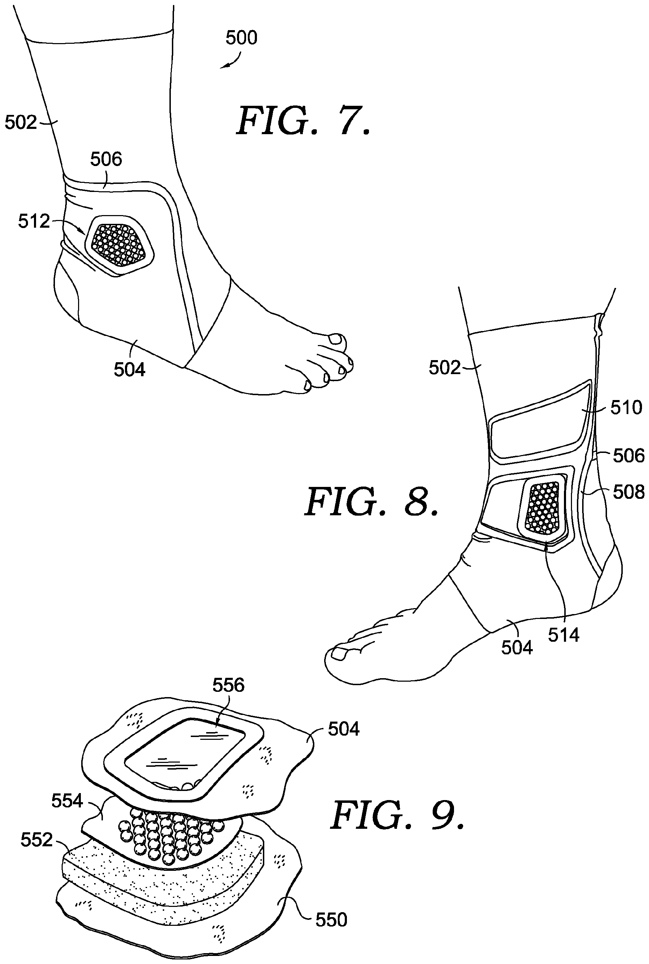

Like the sleeves described above with respect to FIGS. 1-3, the ankle and calf sleeve 400 has a lateral malleolus cushioning pad 418 and a medial malleolus cushioning pad 420. The structure of pads 418 and 420 is further described below with respect to FIG. 9. The material used for sleeves 402 and 404 can be any of a number of woven materials typically used for compression-type sleeves, such as a woven nylon material with elastic properties. The stitch lines 406 and 408 join the upper sleeve 402 and lower sleeve 404 but are positioned such that there are no seams in the area of foot striking (such as occurs during soccer) or on the underside of the foot. This construction allows woven materials to be used, along with a stitch-and-sew construction, without compromising the comfort of the athlete.

A sleeve 500, similar to sleeve 400, is shown in FIGS. 7 and 8. Sleeve 500 differs in that it extends only slightly above the ankle region, as opposed to the added protection of sleeve 400. As shown in FIGS. 7 and 8, sleeve 500 includes an upper sleeve 502 and a lower sleeve 504 joined together along stitch lines 506 and 508. As with sleeve 400, stitch lines 506 and 508 are positioned such that the foot-striking zone, as well as the underside of the foot, remains free from seams to enhance the comfort of the athlete. Sleeves 502 and 504 are made from the same or similar materials as sleeves 402 and 404 described above. Upper sleeve 502 includes a medial cushioning pad 510 that is held in place in the same fashion as pads 410-414 described above. Sleeve 500 further includes a lateral malleolus cushioning pad 512 and a medial malleolus cushioning pad 514.

The layered construction of the area around cushioning pads 418, 420, 512, and 514 is shown in an exploded view in FIG. 9. As shown, an underlying base fabric 550 is disposed on the inner side, facing the athlete when worn. Fabric 550 can be the same as that used for sleeves 402, 404, 502, and 504, or could be a no-shrink mesh material that helps maintain the desired shape even when washed or saturated. The fabric 550 is covered by a foam material 552 that can be the same as that used for cushioning pads 410-414 and 510. Preferably, an abrasion and puncture-resistant material 554 is disposed over foam material 552. One exemplary material 554 is a composition of ceramic particles anchored in a polymer matrix such as the CERASPACE™ fabric available from the Schoeller Textiles of Switzerland. Material 554 may be covered with an optional windowing layer 556 that allows a view of material 554. However, the windowing layer could be eliminated and the material 554 could be covered with the outer fabric layer such as that used for sleeves 402, 404, 502, and 504. The sleeves of FIGS. 4-9 provide an ankle and calf sleeve, or an ankle sleeve, with many of the same properties of the sleeves of FIGS. 1-3 but provide a stitch-and-sew construction that allows a wider variety of materials to be used.

While the concepts provided herein discuss the concept of a protective gear and depict a strategically cushioned ankle and foot sleeve in particular, it is contemplated that this concept extends to all types of impact protective gear. For example, features provided herein may be utilized in connection with helmets, clothing, barriers, armor, and other applications.

From the foregoing, it will be seen that the disclosed protective gear is well adapted to attain all the ends and objects hereinabove set forth together with other advantages, which are obvious and inherent to the structure.

It will be understood that certain features and subcombinations are of utility and may be employed without reference to other features and subcombinations. This is contemplated by and is within the scope of the claims.

Since many possible alternative protective garments may be made without departing from the scope thereof, it is to be understood that all matter herein set forth or shown in the accompanying drawings is to be interpreted as illustrative and not in a limiting sense, the scope is defined by the appended claims only.

## Claims

1. A foot and ankle protective garment (100, 200, 300), comprising: a knitted elastic sleeve portion (150, 350) having an inner and an outer surface configured to wrap around at least a portion of a user's foot and a portion of a user's ankle; at least one medial malleolus cushioning element (130, 131, 132, 330) attached to the outer surface of the elastic sleeve portion (150, 350) such that a medial malleolus of a user is protected from impact; at least one lateral malleolus cushioning element (120, 320) attached to the outer surface of the elastic sleeve portion (150, 350) such that a lateral malleolus of a user is protected from impact; and a lower shin area cushioning element (140, 340) attached to the outer surface of the elastic sleeve portion (150, 350) such that a lower shin area of a user is protected from impact, wherein the shin area cushioning element (140, 340) includes a vertically oriented slit (142, 342) extending from an open upper end, located on a top side of the lower shin area cushioning element (140, 340), to a closed lower end, for easy donning of the foot and ankle protective garment, wherein the slit (142, 342) allows the knitted sleeve to stretch beyond the stretchability of the lower shin cushioning element (140, 340) to an open position, and after donning of the garment (100, 200, 300) onto a user's foot and ankle, the slit (142, 342) returns to its starting position and is substantially closed, thereby offering a nearly continuous protective pad that will protect the lower shin area of the user.

2. The protective garment (100, 200, 300) of claim 1, wherein the lower shin area cushioning element slit (142, 342) has a length that is at least 30% of the total length of the lower shin area cushioning element (140, 340).

3. The protective garment (100, 200, 300) of claim 1, further comprising an Achilles tendon cushioning element (110) attached to the outer surface of the elastic sleeve portion (150, 350) such that an Achilles tendon of a user is protected from impact.

4. The protective garment (100, 200, 300) of claim 3, wherein the Achilles tendon cushioning element (110), the at least one medial malleolus cushioning element (130, 131, 132, 330), the at least one lateral malleolus cushioning element (120, 320) and the lower shin area cushioning element (140, 340) are formed from an impact-absorbing foam.

5. The protective garment (100, 200, 300) of claim 1, further comprising a protective shell (220) disposed over said medial malleolus cushioning element (130, 131, 132, 330) for providing abrasion resistance.

6. The protective garment (100, 200, 300) of claim 1, wherein the elastic sleeve (150, 350) is a circularly knit sleeve.

7. The protective garment (100, 200, 300) of claim 6, wherein the elastic sleeve (150, 350) comprises one or more of spandex fibers, moisture management fibers, and natural fibers.

8. The protective garment (100, 200, 300) of claim 1, further comprising an adaptor element (305) for removably attaching a full-length protective shin guard.

9. The foot and ankle protective garment (100, 200, 300) of claim 1, wherein the medial malleolus cushioning element (130, 131, 132, 330) is multi-layered; the lateral malleolus cushioning element (120, 320) is multi-layered; and the lower shin area cushioning element (140, 340) is multi-layered.

10. The foot and ankle protective garment (100, 200, 300) of claim 9, wherein an upper layer of the medial malleolus cushioning element (130, 131, 132, 330) and an upper layer of the lateral malleolus cushioning element (120, 320) comprise a protective shell for providing abrasion protection.

## Patentansprüche

1. Fuß und Knöchel schützendes Kleidungsstück (100, 200, 300), umfassend: einen gestrickten elastischen Manschettenteil (150, 350) mit einer inneren und einer äußeren Oberfläche, der dafür konfiguriert ist, um mindestens einen Teil eines Fußes eines Benutzers und einen Teil eines Knöchels eines Benutzers gewickelt zu werden; mindestens ein mediales Malleolus-Polsterelement (130, 131, 132, 330), das an der äußeren Oberfläche des elastischen Manschettenteils (150, 350) befestigt ist, so dass ein medialer Malleolus eines Benutzers vor Stößen geschützt ist; mindestens ein laterales Malleolus-Polsterelement (120, 320), das an der äußeren Oberfläche des elastischen Manschettenteils (150, 350) befestigt ist, so dass ein lateraler Malleolus eines Benutzers vor Stößen geschützt ist; und ein Unterschienbeinbereich-Polsterelement (140, 340), das an der äußeren Oberfläche des elastischen Manschettenteils (150, 350) befestigt ist, so dass ein Unterschienbeinbereich eines Benutzers vor Stößen geschützt ist, worin das Schienbeinbereich-Polsterelement (140, 340) einen vertikal orientierten Schlitz (142, 342) beinhaltet, der sich von einem offenen oberen Ende, das sich an einer Oberseite des Unterschienbeinbereich-Polsterelements (140, 340) befindet, zu einem geschlossenen unteren Ende, zum einfachen Anlegen des den Fuß und Knöchel schützenden Kleidungsstücks, erstreckt, worin der Schlitz (142, 342) es der gestrickten Manschette erlaubt, sich über die Dehnbarkeit des Unterschienbein-Polsterelements (140, 340) hinaus in eine offene Position zu dehnen und, nach Anlegen des Kleidungsstücks (100, 200, 300) an einem Fuß und Knöchel eines Benutzers, der Schlitz (142, 342) in seine Ausgangsposition zurückkehrt und im Wesentlichen geschlossen ist, wodurch ein nahezu durchgehendes Schutzpolster geboten wird, das den Unterschienbeinbereich des Benutzers schützt.

2. Schützendes Kleidungsstück (100, 200, 300) nach Anspruch 1, worin der Schlitz (142, 342) des Unterschienbeinbereich-Polsterelements eine Länge aufweist, die mindestens 30 % der Gesamtlänge des Unterschienbeinbereich-Polsterelements (140, 340) ist.

3. Schützendes Kleidungsstück (100, 200, 300) nach Anspruch 1, ferner umfassend ein Achillessehnen-Polsterelement (110), das an der äußeren Oberfläche des elastischen Manschettenteils (150, 350) befestigt ist, so dass eine Achillessehne eines Benutzers vor Stößen geschützt ist.

4. Schützendes Kleidungsstück (100, 200, 300) nach Anspruch 3, worin das Achillessehnen-Polsterelement (110), das mindestens eine mediale Malleolus-Polsterelement (130, 131, 132, 330), das mindestens eine laterale Malleolus-Polsterelement (120, 320) und das Unterschienbeinbereich-Polsterelement (140, 340) aus einem stoßabsorbierenden Schaum gebildet sind.

5. Schützendes Kleidungsstück (100, 200, 300) nach Anspruch 1, ferner umfassend eine Schutzhülle (220), die über dem medialen Malleolus-Polsterelement (130, 131, 132, 330) angeordnet ist, um für Abriebfestigkeit zu sorgen.

6. Schützendes Kleidungsstück (100, 200, 300) nach Anspruch 1, worin die elastische Manschette (150, 350) eine rundgestrickte Manschette ist.

7. Schützendes Kleidungsstück (100, 200, 300) nach Anspruch 6, worin die elastische Manschette (150, 350) eines oder mehrere von Spandex-Fasern, feuchtigkeitsregelnden Fasern und Naturfasern umfasst.

8. Schützendes Kleidungsstück (100, 200, 300) nach Anspruch 1, ferner umfassend ein Adapterelement (305) zum lösbaren Befestigen eines volllangen Schienbeinschützers.

9. Fuß und Knöchel schützendes Kleidungsstück (100, 200, 300) nach Anspruch 1, worin das mediale Malleolus-Polsterelement (130, 131, 132, 330) mehrlagig ist; das laterale Malleolus-Polsterelement (120, 320) mehrlagig ist; und das Unterschienbeinbereich-Polsterelement (140, 340) mehrlagig ist.

10. Fuß und Knöchel schützendes Kleidungsstück (100, 200, 300) nach Anspruch 9, worin eine obere Lage des medialen Malleolus-Polsterelements (130, 131, 132, 330) und eine obere Lage des lateralen Malleolus-Polsterelements (120, 320) eine für Abriebschutz sorgende Schutzhülle umfassen.

## Revendications

1. Vêtement de protection de cheville et de pied (100, 200, 300), comprenant :
une partie manchon élastique tricoté (150, 350) ayant une surface extérieure et une surface extérieure configurées pour entourer au moins une partie d'un pied d'un utilisateur et une partie d'une cheville d'un utilisateur ;
un ou plusieurs éléments d'amortissement de malléole médiane (130, 131, 132, 330) assujettis à la surface extérieure de la partie manchon élastique (150, 350) de telle sorte qu'une malléole médiane d'un utilisateur est protégée des chocs ;
un ou plusieurs éléments d'amortissement de la malléole latérale (120, 320) assujettis à la surface extérieure de la partie manchon élastique (150, 350) de telle sorte qu'une malléole latérale d'un utilisateur est protégée des chocs ; et
un élément d'amortissement de la zone tibia inférieure (140, 340) assujetti à la surface extérieure de la partie manchon élastique (150, 350) de telle sorte qu'une zone tibia inférieure d'un utilisateur est protégée des chocs,
dans lequel l'élément d'amortissement de la zone tibia (140, 340) inclut une fente orientée verticalement (142, 342) s'étendant depuis une extrémité supérieure ouverte située sur une partie supérieure de l'élément d'amortissement de la zone tibia inférieure (140, 340) à une extrémité inférieure fermée, pour faciliter l'enfilement du vêtement de protection de cheville et de pied, dans lequel la fente (142, 342) permet au manchon tricoté de s'étirer au-delà de l'extensibilité de l'élément d'amortissement du tibia inférieur (140, 340) à une position ouverte et, après l'enfilement du vêtement (100, 200, 300) sur une cheville et pied d'un utilisateur, la fente (142, 342) revient à sa position de départ et est sensiblement fermée, offrant ainsi un coussin de protection presque continu qui protégera la zone tibia inférieure de l'utilisateur.

2. Vêtement de protection (100, 200, 300) selon la revendication 1, dans lequel l'élément d'amortissement de la fente de la zone tibia inférieure (142, 342) a une longueur qui fait au moins 30 % de la longueur totale de l'élément d'amortissement de la zone tibia inférieure (140, 340).

3. Vêtement de protection (100, 200, 300) selon la revendication 1, comprenant en outre un élément d'amortissement du tendon d'Achille (110) assujetti à la surface extérieure de la partie manchon élastique (150, 350) de telle sorte qu'un tendon d'Achille d'un utilisateur est protégé des chocs.

4. Vêtement de protection (100, 200, 300) selon la revendication 3, dans lequel l'élément d'amortissement du tendon d'Achille (110), le ou les éléments d'amortissement de malléole médiane (130, 131, 132, 330), le ou les éléments d'amortissement de la malléole latérale (120, 320) et l'élément d'amortissement de la zone tibia inférieure (140, 340) sont formés dans une mousse absorbant les chocs.

5. Vêtement de protection (100, 200, 300) selon la revendication 1, comprenant en outre une coque protectrice (220) disposée sur ledit élément d'amortissement de malléole médiane (130, 131, 132, 330) pour fournir une résistance à l'abrasion.

6. Vêtement de protection (100, 200, 300) selon la revendication 1, dans lequel le manchon élastique (150, 350) est un manchon tricoté circulairement.

7. Vêtement de protection (100, 200, 300) selon la revendication 6, dans lequel le manchon élastique (150, 350) comprend des fibres parmi des fibres d'élasthanne, des fibres régulant l'humidité et des fibres naturelles.

8. Vêtement de protection (100, 200, 300) selon la revendication 1, comprenant en outre un élément adaptateur (305) permettant d'assujettir de manière amovible un protège-tibia de protection de longueur totale.

9. Vêtement de protection de cheville et de pied (100, 200, 300) selon la revendication 1, dans lequel
l'élément d'amortissement de malléole médiane (130, 131, 132, 330) a plusieurs couches ;
l'élément d'amortissement de la malléole latérale (120, 320) a plusieurs couches ; et
l'élément d'amortissement de la zone tibia inférieure (140, 340) a plusieurs couches.

10. Vêtement de protection de cheville et de pied (100, 200, 300) selon la revendication 9, dans lequel une couche supérieure de l'élément d'amortissement de malléole médiane (130, 131, 132, 330) et une couche supérieure de l'élément d'amortissement de la malléole latérale (120, 320) comprennent une coque protectrice pour fournir une résistance à l'abrasion.
